# EUROPEAN PATENT APPLICATION

(11) **EP 4 108 758 A1**
(43) Date of publication of application: **28.12.2022**
(21) Application number: 21756420.2
(22) Date of filing: 15.02.2021
(51) Int. Cl.: C12M 1/00, C12N 5/00

(54) **CELL CULTURE PROCESS SEARCH METHOD, CELL CULTURE PROCESS SEARCH PROGRAM, CELL CULTURE PROCESS SEARCH DEVICE, AND LEARNED MODEL**

(30) Priority: 19.02.2020 JP 2020026428
(71) Applicant: FUJIFILM Corporation, Tokyo 106-8620 (JP)
(72) Inventor: TERAO, Takahiro, Ashigarakami-gun, Kanagawa 258-8538 (JP)
(74) Representative: Klunker IP Patentanwälte PartG mbB
(86) International application number: PCT/JP2021/005417
(87) International publication number: WO 2021/166824

(57) **Abstract**

Provided are a cell culture process search method, a cell culture process search program, and a cell culture process search device capable of searching for an optimal culture condition. A cell culture process search method includes a process condition generation step of generating a plurality of process conditions for culturing a cell, a culture result prediction step of acquiring a culture prediction result of the cell for each of the plurality of process conditions generated in the process condition generation step, and an optimized process condition acquisition step of finding out an optimal process condition from the culture prediction result obtained in the culture result prediction step. A cell culture process search program to be executed and a cell culture process search device are provided. The process condition includes at least either of a plurality of culture medium compositions or a plurality of culture conditions.

## Description

### BACKGROUND OF THE INVENTION

### 1. Field of the Invention

The present invention relates to a cell culture process search method, a cell culture process search program, a cell culture process search device, and a learned model and particularly relates to a cell culture process search method, a cell culture process search program, a cell culture process search device, and a learned model that search for an optimal cell culture process for producing bioproduction that produces a chemical product by using action of a cell, a microorganism, or an enzyme derived from an animal and a plant.

### 2. Description of the Related Art

In examining culture conditions (culture medium, culture method, period, and the like) for bioproduction production, dozens of conditions from a huge number of combinations of the culture conditions are selected for an experiment and the experiment is repeated a plurality of times. A method using an experimental design method is known as a method of selecting the dozens of conditions. However, in the experimental design method, only a limited number of combinations from a huge number of combinations are searched for and additionally a lot of costs (time, labor, materials, and the like) are required due to the repetition of the experiment.

Thus, an efficient search for an optimal condition by a computer is performed. In recent years, research on an analysis of a metabolic circuit has been developed for the purpose of controlling metabolism such as fermentation. In order to search for the optimal culture condition by computer, it is necessary to measure a structure of the metabolic circuit and a constant and the like related to each reaction. However, it is difficult to measure the constant related to a reaction in a cell except for some model organisms. For example, the JP2007-47994A below is known as a method of examining the culture conditions for bioproduction production from the metabolism that can be calculated by using a partially known constant.

On the other hand, a method of knowing biological behavior, such as an amount of metabolic reaction and proliferation, only from the structure of the metabolic reaction circuit has been developed, without using the information on the reaction constant. In flux balance analysis (FBA), even in a case where the constant related to the metabolism cannot be sufficiently measured, a behavior range of a target metabolic circuit and characteristics thereof are analyzed based on a basic constraint condition, such as the law of conservation of mass, by using only a structure of a metabolic reaction. For example, in Shilling, C. and Palsson, B., Proc. Nat. Sci. Acad., 95, 4193-4198, 1988 below, the metabolic reaction is first described as a series of linear equations, vector space of a solution of the simultaneous equations is defined, then this vector space is converted into a biochemically meaningful basis, and finally, a metabolic state that maximizes an objective function provided by linear programming is specified.

Further, JP2003-180400A is known as a method of knowing optimal metabolic circuit information by associating FBA with various environmental factors affecting the metabolism, a culture time, and the like. For example, US2012/0191434A and US2012/0185226A are known as a method of predicting a culture result in consideration of time variation and a method of obtaining information for modification into an optimal cell, using FBA. JP2014-503220A is known as a method of optimizing a culture medium by indirectly inferring cell metabolism information from a change in culture medium composition during culture and calculating a necessary component.

### SUMMARY OF THE INVENTION

However, the method described in JP2007-47994A is limited to a target having a relatively small-scale such as a microorganism or some biological functions. The method described in Shilling, C. and Palsson, B., Proc. Nat. Sci. Acad., 95, 4193-4198, 1988 does not consider any information regarding the culture such as culture time and culture composition. The methods described in JP2003-180400A to 4 are not methods for knowing the culture prediction result in consideration of time variation, the optimal culture condition, and the culture medium composition. The method described in JP2014-503220A is not a method of searching for the optimal culture condition and the culture medium composition without conducting an experiment.

As described above, in the search for the optimal cell culture process by a computer so far, a search method in consideration of the culture medium composition, the culture method, the culture period, and the like has not been performed.

The present invention has been made in view of such circumstances and provides a cell culture process search method, a cell culture process search program, a cell culture process search device, and a learned model capable of searching for an optimal culture condition with an overall view of a culture process without repeating an experiment for cell culture and bioproduction production.

In order to achieve an object of the present invention, a cell culture process search method includes a process condition generation step of generating a plurality of process conditions for culturing a cell, a culture result prediction step of acquiring a culture prediction result of the cell for each of the plurality of process conditions generated in the process condition generation step, and an optimized process condition acquisition step of finding out an optimal process condition from the culture prediction result obtained in the culture result prediction step.

In one aspect of the present invention, the process condition includes at least either of a plurality of culture medium compositions or a plurality of culture conditions.

In one aspect of the present invention, the culture condition is preferably a setting condition including at least any one of a culture method, a size and type of a culture vessel, oxygen addition, replenishment of a culture medium and a nutrient, removal of a culture medium containing a proliferation-inhibiting by-product, or harvesting of a target product.

In one aspect of the present invention, the culture condition preferably includes a design and operation of scale-up from a small-scale process to a large-scale process.

In one aspect of the present invention, the plurality of process conditions generated in the process condition generation step are preferably acquired as a matrix.

In one aspect of the present invention, in the process condition generation step, it is preferable that a part of items of the process condition is selected and a numerical value for the selected item is determined.

In one aspect of the present invention, the process condition generation step is preferably performed by a method including at least any one of a method of determining a numerical value for an item of the process condition based on a predetermined numerical value, a method of performing the determination by random number generation in a predetermined range, a method of performing the determination by a numerical value obtained by an experiment, or a method of performing the determination based on an expansion culture medium mixing strategy.

In one aspect of the present invention, the predetermined range is preferably set by using a mathematically modeled equation of a mechanism by which an organism takes in a culture medium component.

In one aspect of the present invention, the mathematically modeled equation is preferably Michaelis-Menten kinetics or Fick's law.

In one aspect of the present invention, in the method by the random number generation, the numerical value for the item of the process condition is preferably determined by using a numerical value generated by a continuous uniform random number, a continuous normal random number, a discrete random number, or a binary random number.

In one aspect of the present invention, the culture result prediction step preferably includes a mechanism of the cell to be cultured and a cell culture simulation method that reproduces a bioprocess.

In one aspect of the present invention, the cell culture simulation method preferably includes a modeling approach including metabolic flux analysis using a genome-scale metabolic model or flux balance analysis.

In one aspect of the present invention, the optimized process condition acquisition step preferably includes an input step of inputting the process condition generated in the process condition generation step and the culture prediction result acquired in the culture result prediction step, a creation step of creating a learned model by machine learning with the process condition and the culture prediction result, which are input in the input step, as learning data, and a calculation step of calculating an optimal process by solving an inverse problem using the learned model.

In one aspect of the invention, the culture condition is preferably a setting condition including at least any one of a culture method, a size and type of a culture vessel, oxygen addition, replenishment of a culture medium and a nutrient, removal of a culture medium containing a proliferation-inhibiting by-product, or harvesting of a target product.

In one embodiment of the invention, the culture condition preferably includes a design and operation of scale-up from a small-scale process to a large-scale process.

In one aspect of the invention, the cell is preferably a eukaryotic cell or a prokaryotic cell.

In one aspect of the invention, the eukaryotic cell is preferably a cell strain derived from an animal, a plant, or an insect, a primary culture product, or a fungus.

In one aspect of the present invention, the prokaryotic cell is preferably a bacterium including Escherichia coli, Bacillus subtilis, cyanobacteria, or actinomycetes and an archaeon including methanogen, extreme halophile, or hyperthermophile.

In order to achieve the object of the present invention, a cell culture process search program according to the present invention causes a computer to execute the cell culture process search method described above.

In order to achieve the object of the present invention, a cell culture process search device according to the present invention has a process condition generation unit that generates a plurality of process conditions for culturing a cell, a culture result prediction unit that acquires a culture prediction result of the cell for each of the plurality of process conditions generated by the process condition generation unit, and an optimized process condition acquisition unit that finds out an optimal process condition from the culture prediction result obtained by the culture result prediction unit.

In order to achieve the object of the present invention, a learned model according to the present invention is used to calculate an optimal process condition, in which the learned model is obtained by machine learning based on a plurality of process conditions and culture prediction results obtained from the plurality of process conditions.

According to the present invention, since the optimal culture medium composition or the optimal culture condition can be obtained without repeating the experiment, an efficient culture process search is possible.

### BRIEF DESCRIPTION OF THE DRAWINGS

Fig. 1 is a block diagram showing a configuration of a cell culture process search device.
Fig. 2 is a block diagram showing a configuration of a processing unit.
Fig. 3 is a flowchart showing a search method of the cell culture process.
Fig. 4 is a diagram describing a graph for obtaining a numerical value by a random number.
Fig. 5 is a flowchart showing a culture result prediction step.
Fig. 6 is a schematic diagram showing a metabolic pathway of a cell.
Fig. 7 is a simulation calculation flow.
Fig. 8 is a diagram describing an outline of Michaelis-Menten kinetics.
Fig. 9 is a table of results indicated by the culture result prediction step.

### DESCRIPTION OF THE PREFERRED EMBODIMENTS

Hereinafter, a cell culture process search method, a cell culture process search program, and a cell culture process search device according to the present invention will be described with reference to accompanying drawings. First, the cell culture process search device to execute the cell culture process search method of the present embodiment will be described.

### «Cell Culture Process Search Device»

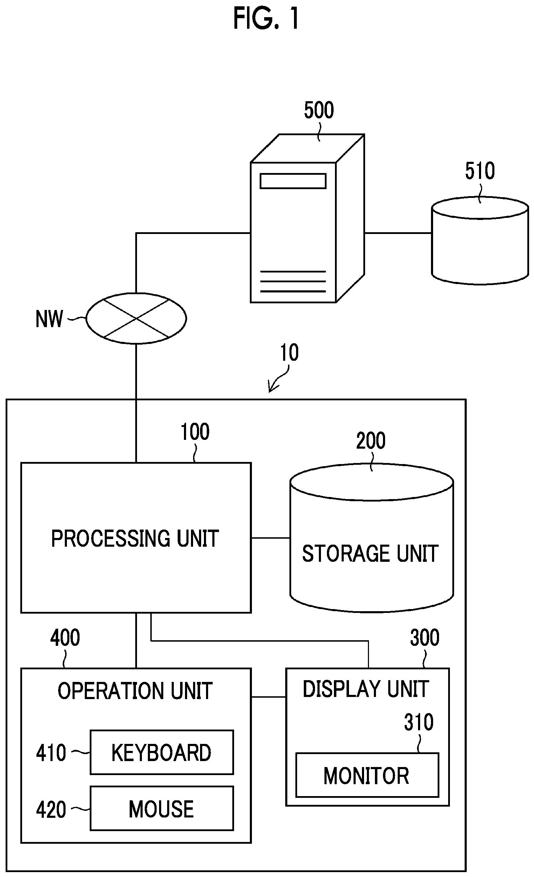

Fig. 1 is a block diagram showing a configuration of a cell culture process search device (hereinafter also simply referred to as "search device") 10. The search device 10 is a device that searches for an optimal process condition from a plurality of process conditions and can be formed by using a computer. As shown in Fig. 1, the search device 10 comprises a processing unit 100, a storage unit 200, a display unit 300, and an operation unit 400 and is connected to transmit and receive necessary information. Various installation forms can be employed for these components. Each component may be installed in one place (inside one housing, one room, or the like) or may be installed in a remote place and connected via a network. Further, the search device 10 is connected to an external server 500 and an external database 510 via a network NW such as the Internet and can acquire information such as a culture medium composition and condition of a culture medium or a condition and mathematical model used for simulation.

### <Configuration of Processing Unit>

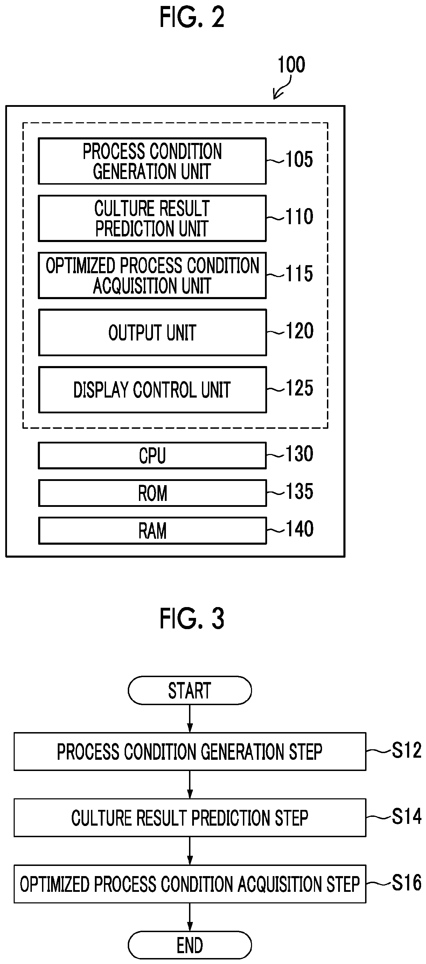

Fig. 2 is a diagram showing a configuration of the processing unit 100. The processing unit 100 comprises a process condition generation unit 105, a culture result prediction unit 110, an optimized process condition acquisition unit 115, an output unit 120, a display control unit 125, a central processing unit 130 (CPU), a read only memory 135 (ROM), and a random access memory 140 (RAM).

The process condition generation unit 105 generates the plurality of process conditions for culturing a cell. The process condition includes at least either of a plurality of culture medium components or a plurality of culture conditions. In generation of the plurality of culture medium components, the culture medium components of the culture medium for culturing the cell and an amount of the culture medium components (composition ratio of the culture medium components) are determined to generate the plurality of culture medium compositions. In the determination of the plurality of culture conditions, the culture conditions for culturing the cell and a numerical value for each culture condition are determined to perform the determination of the plurality of culture conditions. The culture result prediction unit 110 calculates cell culture prediction results by simulation for the process conditions (generated culture medium compositions and determined culture conditions) generated by the process condition generation unit 105. The optimized process condition acquisition unit 115 finds out the optimal process condition (culture medium composition and culture condition) from the culture prediction results obtained by the culture result prediction unit 110.

The output unit 120 outputs the process conditions generated by the process condition generation unit 105. The output unit 120 outputs the culture prediction results of the cell obtained by the culture result prediction unit 110. Further, the output unit 120 outputs the optimal process condition acquired by the optimized process condition acquisition unit 115. The optimized process condition acquisition unit 115 may rearrange the culture prediction results obtained by the culture result prediction unit 110 from the top of good results, and the output unit 120 may output the culture prediction results in the rearranged order. The display control unit 125 controls the display of the acquired information and the processing result on a monitor 310. The cell culture process search method using these functions of the processing unit 100 will be described in detail. The processing by these functions is performed under the control of the CPU 130.

The functions of each part of the processing unit 100 described above can be realized by using various processors. The various processors include, for example, a CPU, which is a general-purpose processor that executes software (program) to realize various functions. The various processors described above also include a programmable logic device (PLD), which is a processor whose circuit configuration can be changed after manufacturing, such as a field programmable gate array (FPGA). Further, the various processors described above also include a dedicated circuitry, which is a processor having a circuit configuration specifically designed to execute specific processing such as an application specific integrated circuit (ASIC), and the like.

The functions of each part may be realized by one processor or may be realized by combining a plurality of processors. A plurality of functions may be realized by one processor. As an example of configuring the plurality of functions by one processor, firstly, there is a form in which one processor is constituted of a combination of one or more CPUs and software and the processor realizes the plurality of functions, as represented by a computer such as a client or a server. Secondly, there is a form in which a processor that realizes functions of the entire system by one integrated circuit (IC) chip is used, as represented by a system on chip (SoC). As described above, a hardware structure for the various functions is constituted of using one or more of the various processors described above. Further, as the hardware structure of the various processors, more specifically, an electric circuit (circuitry) in which circuit elements such as semiconductor elements are combined may be used.

In a case where the above processor or circuitry executes the software (program), a processor (computer) readable code of the executing software is stored in a non-transitory recording medium such as the ROM 135 (refer to Fig. 2) and the processor refers to the software. The software stored in the non-transitory recording medium includes a program for executing the cell culture process search method according to the present invention. The code may be recorded in various magneto-optical storages and a non-transitory recording medium such as a semiconductor memory instead of the ROM 135. In a case where processing using the software is performed, for example, the RAM 140 may be used as a transitory storage region or data stored in an electronically erasable and programmable read only memory (EEPROM) (not shown) may be referred to.

### <Configuration of Storage Unit>

The storage unit 200 is composed of a non-transitory recording medium, such as a digital versatile disk (DVD), a hard disk, or various semiconductor memories, and a control unit thereof. The storage unit stores each component constituting the culture medium composition and each item to determine the culture condition, for example, a culture method, a size and type of a culture vessel, enzyme addition, replenishment of the culture medium and a nutrient, removal of the culture medium containing proliferation-inhibiting by-product, and harvesting of a target product. In addition, the cell culture prediction results calculated by the culture result prediction unit 110 are stored. Further, the optimal process condition obtained by the optimized process condition acquisition unit 115 is stored.

### <Configurations of Display Unit and Operation Unit>

The display unit 300 comprises the monitor 310 (display device) and can display input information, information stored in the storage unit 200, a processing result by the processing unit 100, and the like. The operation unit 400 includes a keyboard 410 and a mouse 420 as input devices and/or pointing devices, and a user can perform necessary operations to execute the cell culture process search method according to the present embodiment through screens of these devices and the monitor 310. The operations that can be executed by the user include inputting a desired cell proliferation amount and a production amount of bioproduction such as an antibody produced by the cell proliferation, inputting the culture medium component or the amount of the culture medium component and the culture condition or the numerical value of the culture condition in a case where the user determines the process condition in the process condition generation unit 105, inputting an output method of the result in the optimized process condition acquisition unit 115, for example, the number of candidate culture media to be output, designation of a mathematical model for determining a taking-in constraint condition of the numerical value in the culture result prediction unit 110, designation of a simulation method to be used, and the like.

### <Process in Cell Culture Process Search Device>

In the cell culture process search device 10 described above, the cell culture process can be searched according to a user's instruction via the operation unit 400.

### «Cell Culture Process Search Method»

Fig. 3 is a flowchart showing the search method of the cell culture process of the present invention. The search method of the cell culture process of the present invention has a process condition generation step in which the plurality of process conditions for culturing the cell are generated, a culture result prediction step in which the cell culture prediction result is acquired for each of the plurality of process conditions generated in the process condition generation step, and an optimized process condition acquisition step in which the optimal process condition is found from the culture prediction results obtained in the culture result prediction step. The process condition includes at least either of the plurality of culture medium components or the plurality of culture conditions.

Hereinafter, each step will be described.

### <Process Condition Generation Step (Step S12)>

The process condition generation unit 105 of the search device 10 performs the process condition generation step (step S12). In the process condition generation step, the plurality of process conditions are generated. The process condition includes at least either of the plurality of culture medium components or the plurality of culture conditions. The generation of the plurality of culture medium compositions is performed by determining the culture medium component of the culture medium for culturing the cell and the amount of the culture medium component. The determination of the plurality of culture conditions is performed by determining the culture conditions for culturing the cell and the numerical value for each culture condition.

Regarding the generation of the culture medium composition, the culture medium for the cell culture is composed of a large number of components (50 or more components in case of culture medium for animal cell). In the generation of the culture medium composition, the culture medium components and the amount of each culture medium component, that is, the composition ratio of the culture medium components are determined.

The amount (composition ratio) of the culture medium components for generating the culture medium composition is preferably determined by a method including any one of a method of performing the determination based on a predetermined numerical value, a method of performing the determination by random number generation in a predetermined range, a method of performing the determination by a numerical value obtained by an experiment, or a method of performing the determination based on an expansion culture medium mixing strategy. As the method of performing the determination based on the expansion culture medium mixing strategy, for example, a method described in Martin Jordan et al., Cytotechnology, 65, 31-40, 2013 can be used. In the generation of the culture medium composition, some culture medium components may be selected by the user and other culture medium components may be selected by the computer. Further, the amount (composition ratio) of the culture medium components selected by the user can be determined. The amount (composition ratio) of the culture medium components selected by the user may be constant or may be changed. All the selection of the culture medium component and the determination of the amount (composition ratio) of the culture medium components may be performed by the computer.

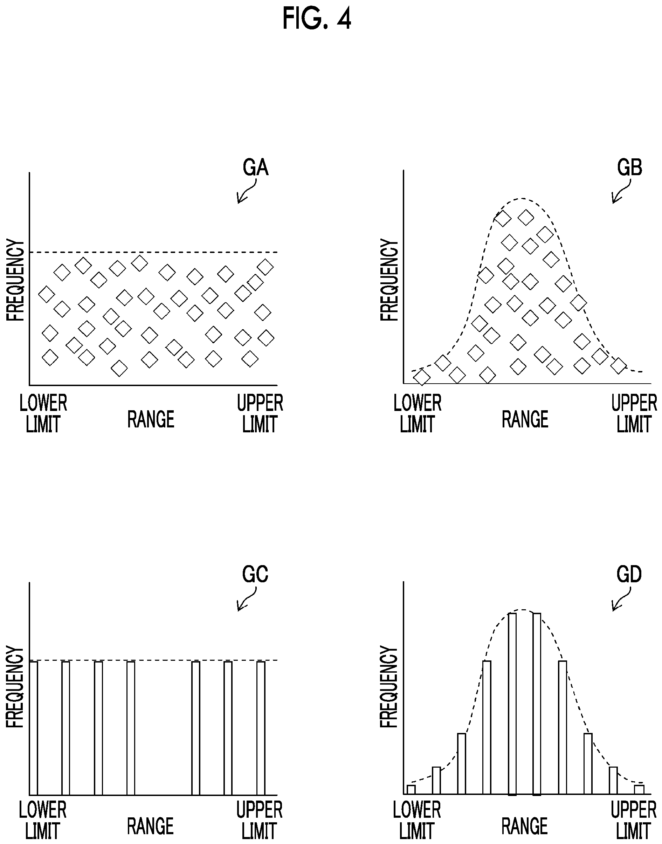

As the method of determining the numerical value of the culture medium composition by the random number generation, the determination can be made by using a continuous uniform random number in which the numerical value is selected with the same probability within a predetermined range as shown in a graph GA in Fig. 4 and a continuous normal random number in which the numerical value is selected based on a normal distribution within a predetermined range as shown in a graph GB. Further, numerical values selected with a predetermined spacing in a predetermined range may be determined by using a discrete random number in which the numerical value is selected with the same probability within the predetermined range as shown in a graph GC. Numerical values selected with a predetermined spacing may be determined by using a binary random number in which the numerical value is selected based on a normal distribution within a predetermined range as shown in a graph GD.

The culture conditions are setting conditions for optimizing the cell culture process, and the setting conditions include items such as the culture method, the size and type of the culture vessel, the oxygen addition, the replenishment of the culture medium and the nutrient, addition of pH adjuster and carbon dioxide, the removal of the culture medium containing proliferation-inhibiting by-product, and the harvesting of the target product. Also in the determination of the culture conditions, as in the case of the culture medium composition, some culture medium conditions may be selected by the user and other culture medium conditions may be selected by the computer. All the determination of the culture conditions and the determination of the numerical values of the culture conditions may be performed by the computer. The numerical values of the culture conditions mean, for example, numerical setting conditions in the culture conditions such as an amount of culture medium, the size of the culture vessel, an amount of additives such as the culture medium and the nutrient, an addition rate of the additive, and a culture temperature.

Examples of the culture method include presence or absence of sterilization treatment, whether the culture medium is liquid or solid, and the culture temperature. The size of the culture vessel may be set randomly and can be determined by the user as appropriate. The culture conditions from a small-scale process to a large-scale process can be determined by changing a size of the culture vessel, and culture results can be predicted by the next culture result prediction step. Accordingly, it is possible to optimize a design and operation of scale-up by simulation and support the culture from a small-scale to a large-scale.

In the culture process, not only conditions at the start of the culture, but also items such as presence or absence of oxygen addition and an addition amount during a culture period, presence or absence of the replenishment of the culture medium and the nutrient and a replenishment amount, addition of pH adjuster and carbon dioxide, presence or absence of the removal of the culture medium containing proliferation-inhibiting by-product and a removal amount, and presence or absence of the harvesting of the target product and a harvesting amount can be set as the culture conditions. The addition amount or removal amount and an addition time or removal time can be set as numerical values of the culture conditions.

In a case where the numerical values for each culture medium component and culture condition are randomly determined by using random numbers, a set range of selected random numbers is preferably set by using a mathematically modeled equation of a mechanism by which an organism takes in the culture medium component. As the mathematically modeled equation, Michaelis-Menten kinetics or Fick's law can be used. The Michaelis-Menten kinetics is an equation indicating a relationship of a reaction rate of an enzymatic reaction with a substrate concentration. A range suitable for the culture medium to be obtained from the beginning can be narrowed down by performing the process condition generation step within a range of Michaelis-Menten kinetics. Therefore, it is possible to reduce the culture medium compositions and culture conditions to be carried out in the next culture result prediction step. The Fick's law is an equation indicating a passing rate in passive diffusion in which a drug molecule passes a cell membrane directly without a carrier such as an enzyme.

The plurality of culture medium compositions generated in the process condition generation step and the plurality of determined culture conditions can be acquired as a culture medium composition matrix and a culture condition matrix, respectively. With the acquisition of the culture medium compositions and the culture conditions as the matrixes, it is possible to check a tendency of the culture prediction results for the culture medium compositions and the culture conditions by reflecting the culture prediction results obtained in the next culture result prediction step in the matrixes.

### <Culture Result Prediction Step (Step S14)>

The culture result prediction unit 110 of the search device 10 performs the culture result prediction step (step S14). In the culture result prediction step, the cell culture prediction results are acquired for the plurality of process conditions generated in the process condition generation step. The culture prediction results include prediction of an amount of bioproduction such as an antibody and an amount of by-product such as ammonia, which are produced by culturing the cell, in addition to prediction of the number of cultured cells.

In the culture result prediction step, the culture prediction results can be obtained by a mechanism of the cell to be cultured and a cell culture simulation method that reproduces a bioprocess. The cell culture simulation method can be performed by a method including a modeling approach including metabolic flux analysis (MFA) using a genome-scale metabolic model or flux balance analysis (FBA).

The "flux" or "metabolic flux" refers to a rate at which a molecule passes a pathway or reaction of interest. Factors that control the flux include a rate of an enzyme catalyst in the pathway, availability (durability) of a substrate, a concentration of the enzyme in the cell, and proximity of the enzyme in the pathway. The "metabolic flux analysis method" is a method of obtaining an amount of moving molecules from these factors. The "flux balance analysis" is an analysis method that focuses on chemical quantity theory and metabolic flux rate. In the flux balance analysis, even in a case where a constant related to the metabolism cannot be sufficiently measured, a behavior range of a target metabolic circuit and characteristics thereof are analyzed based on a basic constraint condition, such as the law of conservation of mass, from a structure of a metabolic reaction.

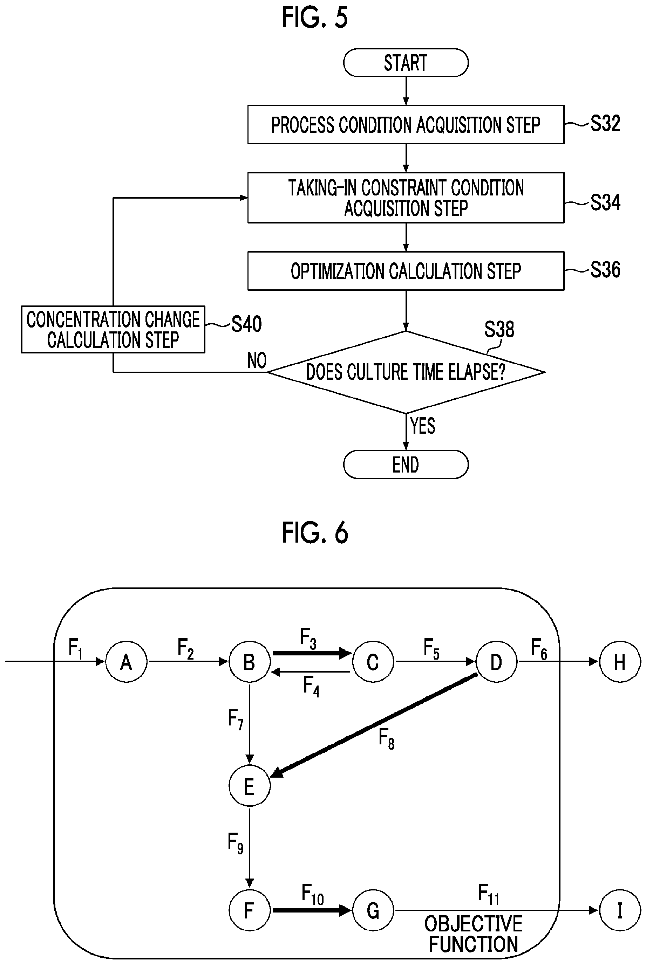

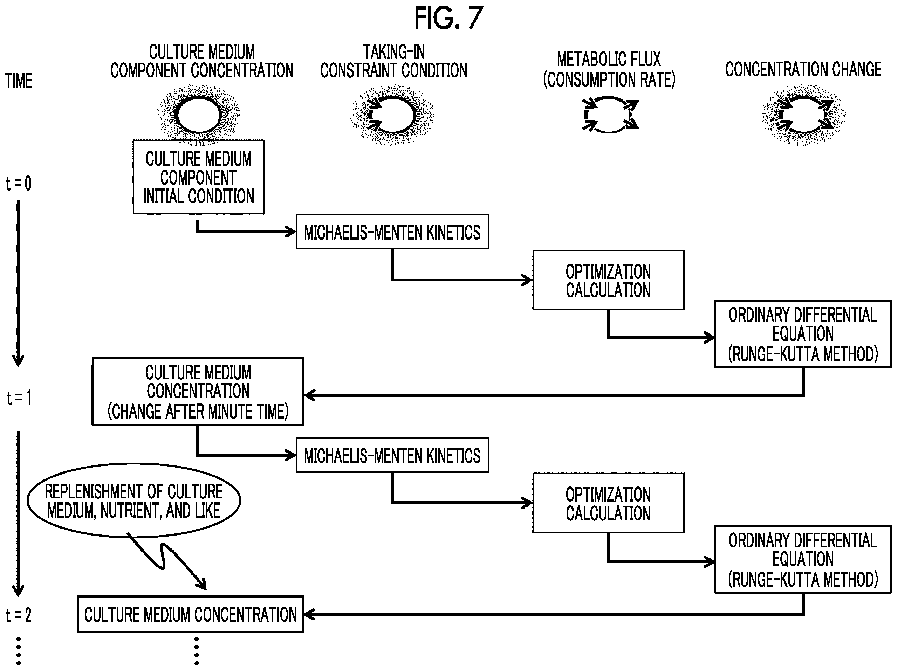

Fig. 5 is a flowchart of the culture result prediction step. Fig. 6 is a schematic diagram showing the metabolic pathway of the cell. Fig. 7 is a simulation calculation flow. The culture result prediction step includes a process condition acquisition step (step S32), a taking-in constraint condition acquisition step (step S34), an optimization calculation step (step S36), and a concentration change calculation step (step S40).

### <Process Condition Acquisition Step (Step S32)>

In the process condition acquisition step, the process condition generated in the process condition generation step (step S12) is acquired.

### <Taking-in Constraint Condition Acquisition Step (Step S34)>

In the taking-in constraint condition acquisition step, the taking-in constraint condition which is upper and lower limits for taking in the substrate, nutrient, and the like used for the metabolism is acquired. In Fig. 6, substances produced by the metabolism are indicated by A, B, C, ··, and the like. Further, F₁, F₂, F₃, ··, and the like are functions indicating a time change of a concentration of each substance. For example, F₁ is a flux that takes in the substance A, and F₂ is a flux that becomes the substance B by the metabolism.

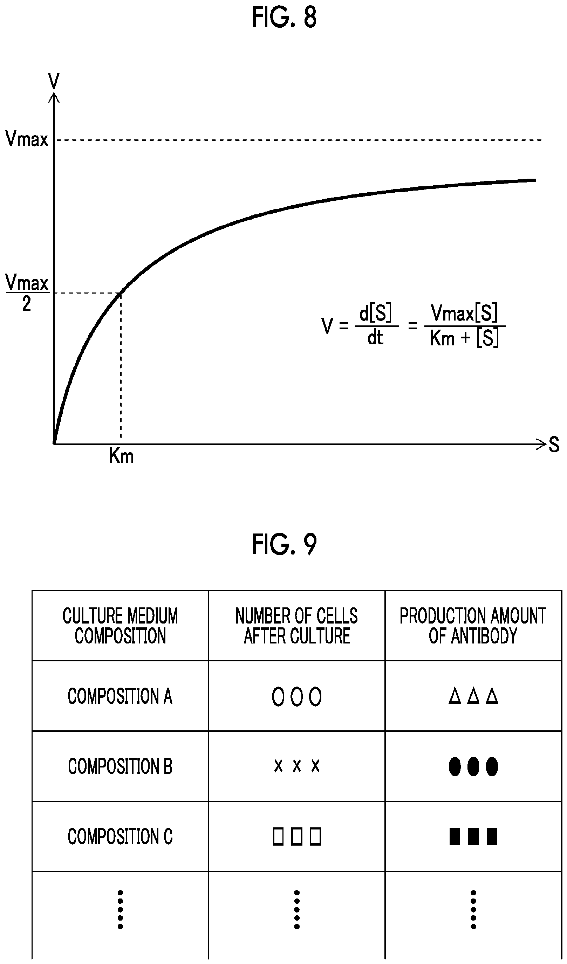

In the example shown in Fig. 6, conversion from the substance A to the substance B is performed in equal amounts according to the law of conservation of mass. Similarly, the substance B has an amount equal to a total amount of conversion into the substance C and the substance E. As described above, the amount of conversion of a substance is determined by amounts of substrate, nutrient, and the like that the cell first takes in. The reaction rate is a parameter that limits the substance conversion. For example, the conversion from the substance A to the substance B is performed in equal amounts as described above, but there is limitation on the reaction rate at a certain unit time. Thus, there is an upper limit to the conversion to substance B, and all the substance A may not be converted to the substance B. In the taking-in constraint condition acquisition step, this constraint condition is acquired by the mathematically modeled equation of the mechanism by which the organism takes in the culture medium component, and the acquired constraint condition is applied in a case where the culture result is calculated. As the mathematical model for obtaining this reaction rate, for example, Michaelis-Menten kinetics can be used. An outline of Michaelis-Menten kinetics is shown in Fig. 8. The Michaelis-Menten kinetics is an equation relating to a reaction rate V of the enzyme. In a case where a substrate concentration S is low, the reaction rate V is proportional to the substrate concentration S, and in a case where the substrate concentration S is high, the reaction rate V converges to a maximum rate Vmax independent of the substrate concentration S. The Fick's law can be additionally used. The Fick's law is an equation for obtaining a flux, which is an amount that passes through a unit area per unit time, and the flux is proportional to a diffusion coefficient D and a substrate concentration gradient on both sides of the membrane. In the present embodiment, with the use of Michaelis-Menten kinetics or Fick's law, the simulation can be performed without calculating a condition that is a reaction rate faster than the reaction rate and flux obtained by the mathematical model, but is within a range of the amount of conversion according to the law of conservation of mass, as a constraint condition for taking in the culture medium. Therefore, it is possible to perform more accurate culture prediction.

### <Optimization Calculation Step (Step S36)>

In the optimization calculation step, a metabolic flux (consumption rate) is calculated by using a mathematical model for cell metabolism (metabolic circuit model) based on the culture medium composition (culture medium component concentration) and the taking-in constraint condition acquired in the process condition acquisition step (step S32) and the taking-in constraint condition acquisition step (step S34). The calculation of the metabolic flux can be performed by, for example, a linear programming method (LP) as shown in Fig. 6. In Fig. 6, a condition that the objective function F₁₁ is maximized is obtained. The F₁₁ is a rate at which the target product is produced and becomes a cell proliferation rate in a case where a desired simulation result is cell proliferation and a by-product producing rate in a case where a desired simulation result is the production amount of bioproduction such as an antibody. With calculation of each of the functions F₁ to F₁₁ that maximize the objective function F₁₁, d[A]/dt = -F₁, d[B]/dt = -F₂, ··, and d[I]/dt = -F₁₁, within a constraint condition range, it is possible to obtain the metabolic flux of each reaction function at a certain time point. This is an optimal metabolic flux (optimization condition) required at a certain time point.

In the optimization calculation step, a plurality of mathematical models that imitate a state of the cell may be arranged in parallel. For example, a mathematical model for the cell proliferation and a mathematical model for producing only the bioproduction without the cell proliferation are arranged in parallel, and a ratio between the plurality of models can be changed according to a culture state such as a culture medium concentration.

In a case where a predetermined culture time (for example, 14 days) for performing the calculation has elapsed (YES) after the optimization calculation step (step S36) ends, the calculation ends. In a case where the predetermined culture time has not elapsed (NO), the next concentration change calculation step is performed (step S38).

### <Concentration Change Calculation Step (Step S40)>

In the concentration change calculation step, a concentration change of the culture medium around the cell in a case where the metabolism is performed under the optimization condition obtained in the optimization calculation step (step S36) is obtained. The concentration change is obtained by solving an ordinary differential equation by a Runge-Kutta method.

The concentration change around the cell after a minute time t has elapsed is obtained in the concentration change calculation step, then the process returns to the taking-in constraint condition acquisition step (step S34) to acquire the taking-in constraint condition of the concentration around the cell after the minute time t has elapsed, and the optimization calculation step (step S36) is performed. Thereafter, the concentration change calculation step (step S40), the taking-in constraint condition acquisition step (step S34), and the optimization calculation step (step S36) are repeated until the predetermined culture time elapses.

In a case where the culture medium, the nutrient, the pH adjuster, and the carbon dioxide are determined to be replenished (hereinafter also referred to as "replenishment of culture medium, nutrient, and the like"), as the culture condition, after a lapse of a predetermined time (replenishment at start of t = 2 in Fig. 7), the calculation is performed by using, as the culture medium composition, a composition concentration obtained by adding the replenished culture medium component to the culture medium concentration around the cell at a time point at which the calculation of t = 1 ends.

In addition to the replenishment of the culture medium, the nutrient, and the like, the removal of the culture medium containing proliferation-inhibiting by-product and the harvesting of the target product may be added as conditions to be performed on the way. In this case, the calculation can be performed by reflecting the replenishment thereof, the removal thereof, and the harvesting thereof in the culture medium composition around the cell in the concentration change calculation step (step S40).

After a predetermined culture period has elapsed (step S38), the calculation ends. At least either of the number of cells proliferated during the culture period or the production amount of the bioproduction, such as an antibody, to be produced or the by-product such as ammonia, to be produced is obtained as a result.

### <Optimized Process Condition Acquisition Step (Step S16)>

The optimized process condition acquisition unit 115 of the search device 10 performs the optimized process condition acquisition step (step S16). In the optimized process condition acquisition step, the optimal process condition is found from the culture prediction results obtained in the culture result prediction step (step S14).

The optimal process condition can be found out by selecting a predetermined number in descending order of the cell proliferation amount or a predetermined number in descending order of the production amount of the bioproduction such as antibody. Results as shown in Fig. 9 are obtained in the culture result prediction step. Among the process conditions generated in the process condition generation step (step S12), the culture prediction results can be obtained for each of the plurality of culture medium compositions and the plurality of culture conditions. With rearrangement of the culture prediction results in descending order of the number of cells cultured or in descending order of the production amount of the bioproduction such as an antibody, it is possible to select the optimal culture medium composition and culture condition.

In the optimized process condition acquisition step, it is possible to find out the optimal process condition by machine learning (deep learning). The optimal process condition includes any one of the optimal culture medium composition or the optimal culture condition. In a case where the machine learning is performed, the plurality of process conditions generated in the process condition generation step and the culture prediction results acquired in the culture result prediction step are input to an input side (input step). Next, with the process conditions and the culture prediction results, which are input in the input step, as learning data, a regression model (learned model) is created by the machine learning (creation step). An inverse problem is solved by using the regression model created as a result of learning to calculate the optimal process condition (calculation step). In a case where the inverse problem is solved, the desired cell proliferation amount, the culture medium composition which is the desired calculated amount of antibody, or the culture condition are calculated. Accordingly, it is possible to acquire the optimal culture medium composition or culture condition among the culture prediction results obtained in the culture result prediction step, using not only results that are ranked high but also results that are not ranked high in a case where the culture prediction results are arranged in descending order of the cell proliferation amount or the production amount of antibody, and thus the calculated culture prediction results can be effectively used.

In the present embodiment, since the culture result is predicted by using the metabolic circuit model, it is possible to obtain information on the flux of the metabolic circuit and a time variation in the prediction process. Since these pieces of information correspond to an action mechanism of the cell culture, it is possible to predict the culture result including the mechanism. Therefore, it is possible to obtain a tendency as to what kind of process condition is required to be selected to obtain a good result, which can be used as a reference in a case where the process condition is determined.

### <Cell Used in Simulation>

The cell used in the cell culture process search method, the cell culture process search program, and the cell culture process search device of the present embodiment is not particularly limited, and both a eukaryotic cell and a prokaryotic cell can be used. As the eukaryotic cell, for example, a cell strain derived from an animal, a plant, or an insect, a primary culture product, or a fungus can be used. As the prokaryotic cell, a bacterium including Escherichia coli, Bacillus subtilis, cyanobacteria, or actinomycetes and an archaeon including methanogen, extreme halophile, or hyperthermophile can be used.

According to the cell culture process search method, the cell culture process search program, and the cell culture process search device of the present invention, the optimal culture medium composition or culture condition can be obtained by simulation. Therefore, it is possible to efficiently search for the culture medium composition or the culture condition without repeating the experiment.

### Examples

Hereinafter, the present invention will be described in more detail with reference to examples of the present invention.

A simulation is performed for the purpose of obtaining the optimal culture medium composition for culturing a CHO cell K1 strain. For metabolic circuit information and model of the CHO cell K1 strain, an FBA model (iCHOv1_K1_final.xml) of the K1 strain is obtained from BiGG Models (http://bigg.ucsd.edu/). Since glucose and 20 kinds of amino acids are used as the culture medium composition, culture data of glucose and 20 kinds of amino acids for the CHO cell K1 strain is obtained from Applied Microbiology and Biotechnology (2015) 99: 4645-4657.

Next, glucose and amino acid taking-in parameters on a model are set by using the above data. As the model to set the parameters, Michaelis-Menten kinetics is used. Next, upper and lower limits of the culture medium concentration are set for glucose and each of 20 kinds of amino acids using the above parameter values to generate 10,000 different compositions (candidate culture media) as random numbers with continuous uniform random numbers. For the generated candidate culture medium, a result for each composition is predicted by the culture simulation incorporating the FBA model, and 10,000 results are obtained. Top 10 results of the above results are selected as candidates for high production culture medium. On the other hand, for 10,000 different results, deep learning is implemented by using the culture medium compositions of glucose and 20 kinds of amino acids as input conditions. New culture medium compositions of 10 pairs are calculated by using a regression model obtained as a result of learning.

It is possible to obtain 20 types of high production culture medium candidates including the previously obtained high production culture medium candidates.

### Explanation of References

- 10:: culture process search device
- 100:: processing unit
- 105:: process condition generation unit
- 110:: culture result prediction unit
- 115:: optimized process condition acquisition unit
- 120:: output unit
- 125:: display control unit
- 200:: storage unit
- 300:: display unit
- 310:: monitor
- 400:: operation unit
- 410:: keyboard
- 420:: mouse
- 500:: external server
- 510:: external database

## Claims

1. A cell culture process search method comprising:
a process condition generation step of generating a plurality of process conditions for culturing a cell;
a culture result prediction step of acquiring a culture prediction result of the cell for each of the plurality of process conditions generated in the process condition generation step; and
an optimized process condition acquisition step of finding out an optimal process condition from the culture prediction result obtained in the culture result prediction step.

2. The cell culture process search method according to claim 1,
wherein the process condition includes at least either of a plurality of culture medium compositions or a plurality of culture conditions.

3. The cell culture process search method according to claim 2,
wherein the culture condition is a setting condition including at least any one of a culture method, a size and type of a culture vessel, oxygen addition, replenishment of a culture medium and a nutrient, removal of a culture medium containing a proliferation-inhibiting by-product, or harvesting of a target product.

4. The cell culture process search method according to claim 3,
wherein the culture condition includes a design and operation of scale-up from a small-scale process to a large-scale process.

5. The cell culture process search method according to any one of claims 1 to 4,
wherein the plurality of process conditions generated in the process condition generation step are acquired as a matrix.

6. The cell culture process search method according to any one of claims 1 to 5,
wherein in the process condition generation step, a part of items of the process condition is selected and a numerical value for the selected item is determined.

7. The cell culture process search method according to any one of claims 1 to 6,
wherein the process condition generation step is performed by a method including at least any one of a method of determining a numerical value for an item of the process condition based on a predetermined numerical value, a method of performing the determination by random number generation in a predetermined range, a method of performing the determination by a numerical value obtained by an experiment, or a method of performing the determination based on an expansion culture medium mixing strategy.

8. The cell culture process search method according to claim 7,
wherein the predetermined range is set by using a mathematically modeled equation of a mechanism by which an organism takes in a culture medium component.

9. The cell culture process search method according to claim 8,
wherein the mathematically modeled equation is Michaelis-Menten kinetics or Fick's law.

10. The cell culture process search method according to any one of claims 7 to 9,
wherein in the method by the random number generation, the numerical value for the item of the process condition is determined by using a numerical value generated by a continuous uniform random number, a continuous normal random number, a discrete random number, or a binary random number.

11. The cell culture process search method according to any one of claims 1 to 10,
wherein the culture result prediction step includes a mechanism of the cell to be cultured and a cell culture simulation method that reproduces a bioprocess.

12. The cell culture process search method according to claim 11,
wherein the cell culture simulation method includes a modeling approach including metabolic flux analysis using a genome-scale metabolic model or flux balance analysis.

13. The cell culture process search method according to any one of claims 1 to 12,
wherein the optimized process condition acquisition step includes
an input step of inputting the process condition generated in the process condition generation step and the culture prediction result acquired in the culture result prediction step,
a creation step of creating a learned model by machine learning with the process condition and the culture prediction result, which are input in the input step, as learning data, and
a calculation step of calculating an optimal process by solving an inverse problem using the learned model.

14. The cell culture process search method according to any one of claims 1 to 13,
wherein the cell is a eukaryotic cell or a prokaryotic cell.

15. The cell culture process search method according to claim 14,
wherein the eukaryotic cell is a cell strain derived from an animal, a plant, or an insect, a primary culture product, or a fungus.

16. The cell culture process search method according to claim 14,
wherein the prokaryotic cell is a bacterium including Escherichia coli, Bacillus subtilis, cyanobacteria, or actinomycetes and an archaeon including methanogen, extreme halophile, or hyperthermophile.

17. A cell culture process search program for causing a computer to execute the cell culture process search method according to any one of claims 1 to 16.

18. A non-transitory computer-readable recording medium on which the cell culture process search program according to claim 17 is recorded.

19. A cell culture process search device comprising:
a process condition generation unit that generates a plurality of process conditions for culturing a cell;
a culture result prediction unit that acquires a culture prediction result of the cell for each of the plurality of process conditions generated by the process condition generation unit; and
an optimized process condition acquisition unit that finds out an optimal process condition from the culture prediction result obtained by the culture result prediction unit.

20. A learned model used to calculate an optimal process condition,
wherein the learned model is obtained by machine learning based on a plurality of process conditions and culture prediction results obtained from the plurality of process conditions.
